# EUROPÄISCHE PATENTSCHRIFT

(11) **EP 2 213 229 B1**
(45) Veröffentlichungstag und Bekanntmachung des Hinweises auf die Patenterteilung: **31.07.2013**
(21) Anmeldenummer: 10005092.1
(22) Anmeldetag: 03.05.2003
(51) Int. Cl.: A61B 5/15

(54) **Blutentnahmesystem**
Blood removal system
Système de prise de sang

(30) Priorität: 28.05.2002 DE 10223558
(43) Veröffentlichungstag der Anmeldung: 04.08.2010
(62) Teilanmeldung aus: 09005455.2
(73) Patentinhaber: Roche Diagnostics GmbH, 68305 Mannheim (DE); F. Hoffmann-La Roche AG, 4070 Basel (CH)
(72) Erfinder: List, Hans, 64754 Hesseneck-Kailbach (DE); Ruschke, Peter, Dr., 55257 Budenheim (DE); Thoes, Bruno, Robert, 66287 Quierschied (DE); Kintzig, Hans, 67311 Tiefenthal (DE); Schabbach, Michael, 69469 Weinheim (DE)
(74) Vertreter: Durm & Partner

(56) Entgegenhaltungen:
- WO-A-01/00090
- US-A- 4 416 279
- US-A- 5 029 583
- US-A- 5 984 940

## Beschreibung

Die Erfindung betrifft ein Blutentnahmesystem zur Entnahme von Blut für Diagnosezwecke.

Um für analytisch-diagnostische Zwecke eine geringe Menge Blut aus einem Körperteil (meistens dem Finger oder dem Ohrläppchen) zu entnehmen, werden Lanzetten verwendet, die zur Erzeugung einer Wunde in das entsprechende Körperteil gestochen werden. Soweit dies manuell geschieht, ist speziell trainiertes Personal erforderlich. Dennoch ist der Einstich mit erheblichem Schmerz verbunden.

Bereits seit langem werden Blutentnahmesysteme verwendet, die aus einem Stechgerät und zugehörigen, für das jeweilige Gerät speziell angepaßten Lanzetten bestehen. In einem Gehäuse des Stechgerätes befindet sich ein Lanzettenantrieb, durch den eine Lanzette mechanisch in die Haut gestochen wird. Als Antriebselement für die Einstichbewegung dient eine Feder. Zu Beginn der Entwicklung waren sehr einfache Konstruktionen gebräuchlich, bei denen die Lanzette unmittelbar an einem Ende einer in einem länglichen Gehäuse angeordneten Druckfeder befestigt war (z.B. US Patent 4,469,110).

Derartige Blutentnahmesysteme wurden jedoch den hohen Anforderungen nicht gerecht, die zu erfüllen sind, wenn eine regelmäßige Überwachung bestimmter analytischer Werte des Blutes erforderlich ist. Dies gilt insbesondere für Diabetiker, die ihren Blutzuckerspiegel häufig kontrollieren sollten, um durch Anpassung von Insulininjektionen an den Bedarf (der abhängig von der Nahrungsaufnahme, der körperlichen Tätigkeit etc. stark schwankt) ihren Blutzuckerspiegel möglichst ständig innerhalb bestimmter Sollgrenzen zu halten. Durch umfangreiche wissenschaftliche Untersuchungen wurde bewiesen, dass mittels einer Intensivtherapie mit mindestens vier Blutanalysen pro Tag ein dramatischer, Rückgang schwerster Spätschäden des Diabetes mellitus (beispielsweise eine Retinopathie mit resultierender Erblindung des Patienten) erreichtwerden kann

Diese Intensivtherapie setzt voraus, dass die Blutentnahme mit einem möglichst geringen Schmerz verbunden ist. Mit dem Ziel, diesbezüglich eine Verbesserung zu erreichen, wurden zahlreiche unterschiedliche Btutentnahmesysteme entwickelt.

Eine schmerzarme Blutentnahme ermöglichen Blutentnahmesysteme, deren Lanzettenantrieb einen Antriebsrotor einschließt, der einerseits (antriebsseitig) mit der Antriebsfeder derartig gekoppelt ist, dass er von dieser in eine Drehbewegung um eine Rotationsachse versetzt werden kann, und der andererseits (abtriebsseitig) über einen Kopplungsmechanismus derartig mit der Lanzette gekoppelt ist, dass die aus der Entspannungsbewegung der Antriebsfeder resultierende Rotation des Antriebsrotors in eine Einstichbewegung umgesetzt wird, bei der die Lanzette mit hoher Geschwindigkeit in Einstichbewegung bewegt wird, bis ihre Spitze aus der Austrittsöffnung austritt, um eine Wunde in einen Körperteil zu erzeugen, das gegen eine die Austrittsöffnung umgebende Kontaktfläche gedrückt wird. Dabei wird die Lanzette von einer Lanzettenführung auf einem vorbestimmten (in der Praxis geraden) Einstichweg geführt.

Eine Blutlanzettenvorrichtung mit einem solchen Rotorantrieb wird in dem US-Patent 4,924,879 beschrieben. Dabei wird der Rotor durch eine koaxiale Spiralfeder angetrieben. Die Drehbewegung des Rotors wird über einen Pleuelantrieb in die erforderliche Linearbewegung der Lanzette umgesetzt.

In dem US-Patent 5,318,584 ist ein Blutentnahmesystem beschrieben, das ebenfalls mit einem Rotorantrieb arbeitet, in großem Umfang (vor allem von Diabetikern) verwendet und wegen seiner unübertroffenen Schmerzarmut sehr geschätzt wird. Der Antriebsrotor dieses Systems rotiert um eine Rotationsachse, die mit der Längsachse des langgestreckten, "pencil-förmigen" Gerätes übereinstimmt. Zum Antrieb dient eine mit dem Rotor koaxiale Drehfeder. Der abtriebsseitige Kopplungsmechanismus zur Umsetzung der Rotationsbewegung in die Translationsbewegung der Lanzette wird von einer Kurvensteuerung gebildet. Die Gestaltung der Steuerkurve ermöglicht es, das Gerät zu spannen, ohne dass die Lanzettenspitze aus dem Gehäuse austritt. Die Rotation des Rotorteils um die Längsachse des Gerätes führt zu einer hohen Vibrationsarmut und stabilisiert den Einstichvorgang. Eine neuere Version eines Blutentnahmesystems mit einem um die Gerätelängsachse rotierenden Antriebsrotor ist in der EP 1034740 A1 beschrieben.

Eine weitere Ausgestaltung eines Rotorantriebs ist in der EP 1090584 A2 beschrieben, bei der ein Antriebsrotor verwendet wird, der um eine quer zu der Einstichrichtung verlaufende Achse rotiert. Die Drehung des Antriebsrotors wird dadurch bewirkt, dass die Kraft der Antriebsfeder gegen eine speziell geformte Druckfläche des Rotors drückt. Dadurch ist es möglich, dass der Rotor sowohl beim Spannen als auch beim Entspannen des Lanzettenantriebs in die gleiche Rotationsrichtung dreht. Auch in diesem Fall schließt der abtriebsseitige Kopplungsmechanismus vorzugsweise eine Kurvensteuerung ein. Die Konstruktion erfordert weniger Bauteile als die zuvor diskutierten Rotorantriebe. Sie bedingt jedoch eine relativ breite Gehäuseform, die von vielen Benutzern als ungünstig angesehen wird.

Trotz der umfangreichen Entwicklungsarbeiten, die zu den vorstehend erörterten und zahlreichen weiteren Konstruktionen geführt haben, besteht ein großes Interesse an einem Blutentnahmesystem, das die schwierigen und teilweise gegenläufigen Anforderungen (minimales Schmerzempfinden, einfache Bedienung, kompakte, möglichst schlanke Bauweise und einfache, kostengünstige Konstruktion) gleichzeitig möglichst weitgehend erfüllt.

Zur Erfüllung dieses Bedürfnisses geht die vorliegende Erfindung von einem Blutentnahmesystem mit einem Rotorantrieb aus. Insoweit kann - insbesondere hinsichtlich des zur Umsetzung der Rotationsbewegung des Antriebsrotors in die Translationsbewegung der Lanzetten erforderlichen abtriebsseitigen Kopplungsmechanismus - auf die vorstehend diskutierten Dokumente verwiesen werden, deren Inhalt durch Bezugnahme zum Gegenstand der vorliegenden Anmeldung gemacht wird. Hiervon ausgehend wird erfindungsgemäß vorgeschlagen, dass sich das von dem Antriebsrotor abgewandte Ende der Antriebsfeder gegen ein drehbewegliches Spannelement abstützt, das Spannelement zum Spannen der Antriebsfeder bei gehemmter Rotation des Antriebsrotors in die gleiche Drehrichtung drehbar ist, in die sich der Antriebsrotor während der Vortriebsphase dreht, und das Spannelement während der Vortriebsphase gegen eine Rückwärtsdrehung arretiert ist, so dass der Antriebsrotor nach Freigabe der Hemmung eine Drehbewegung durchführt, die mittels des abtriebsseitigen Kopplungsmechanismus in die Einstichbewegung der Lanzette umgesetzt wird.

Bei den bisher bekannten Rotorantrieben wurde in der Spannphase meist die Antriebsfeder (und somit der Rotorantrieb insgesamt) dadurch in den gespannten Zustand gebracht, dass der Antriebsrotor (entgegen seiner Drehrichtung während der Einstichbewegung) zurückgedreht wurde. Eine Ausnahme bildet die EP 1090584 A2, bei der die speziell geformte Druckfläche alternierend Vortriebsabschnitte und Spannabschnitte aufweist, so dass die Antriebsfeder bei fortgesetzter, gleichsinniger Rotation des Antriebsrotors gespannt wird, wenn sie in der Spannphase des Rotorantriebs mit einem Spannabschnitt der Druckfläche in Kontakt steht, während sich die Feder in der Vortriebsphase des Antriebs bei Kontakt mit einem Vortriebsabschnitt die Feder entspannt und dadurch die Drehbewegung angetrieben wird.

Bei der Erfindung wird alternierend das drehbewegliche Spannelement und der Antriebsrotor in die gleiche Drehrichtung gedreht:
- Während der Spannphase wird das Spannelement gedreht, während gleichzeitig die Rotation des Antriebsrotors gehemmt ist.
- Während der Vortriebsphase ist das Spannelement gegen eine Rückwärtsdrehung fixiert, so dass der Antriebsrotor nach Freigabe der Hemmung eine Drehbewegung durchführen kann, die mittels des abtriebsseitigen Kopplungsmechanismus in eine entsprechende Translationsbewegung der Lanzette umgesetzt wird

Dieses Prinzip wird nachfolgend als "One Way Alternating Drive And Cocking", kurz OWADAC, bezeichnet. Im Rahmen der Erfindung wurde festgestellt, dass mit der Verwendung dieses Prinzips bei dem Rotorantrieb eines Blutlanzettensystems mehrere bedeutende Vorteile verbunden sind:
- Da der Antriebsrotor in der Spannphase des Lanzettenantriebs nicht bewegt werden muss, tritt die Spitze der Lanzette während des Spannens nicht aus dem Gehäuse aus. Die in dem US-Patent 5,318,584 beschriebene aufwendige Gestaltung der Steuerkurve des abtriebsseitigen Kopplungsmechanismus ist damit nicht notwendig. Es wird eine erhöhte Zuverlässigkeit erreicht.
- Der maximale Drehwinkel des Antriebsrotors ist nicht durch die Erfordernisse des Spannvorgangs limitiert. Deswegen kann der gesamte Drehwinkel von 360 Grad für die Funktion des Blutentnahmesystems genutzt werden. Hieraus resultiert eine verbesserte Relation des Drehwegs des Antriebsrotors zu dem Translationsweg der Lanzette. Dies wiederum ermöglicht eine reibungsärmere Konstruktion des abtriebsseitigen Kopplungsmechanismus, einen schnelleren Ablauf der Drehbewegung in der Vortriebsphase, eine verminderte Vibration während des Einstichvorgangs und eine Reduktion des Schmerzes.
- Der erhöhte Drehwinkelbereich des Antriebsrotors ermöglicht außerdem zusätzliche Vorbereitungsfunktionen vor der eigentlichen Vortriebsphase, beispielsweise zur Kopplung des Lanzettenantriebs mit der Lanzette, wie nachfolgend noch näher erläutert wird.

In der bevorzugten Ausgestaltung der Erfindung stützt sich die Antriebsfeder ohne zwischengeschaltete weitere Bauteile einerseits unmittelbar gegen den Antriebsrotor und andererseits unmittelbar gegen das Spannelement ab. Dies ist zur Vermeidung unnötiger Bauteile und Reibungsverluste zweckmäßig, insbesondere wenn - ebenfalls gemäß bevorzugter Ausführungsform - die Drehachse des Spannelementes koaxial zu der Drehachse des Antriebsrotors verläuft. Grundsätzlich besteht jedoch auch die Möglichkeit, dass die Drehachsen des Spannelementes und des Antriebsrotors zwar parallel, aber nicht koaxial sind, oder dass sie sogar unter einem von Null verschiedenen Winkel zueinander verlaufen. Insbesondere in einem solchen Fall ist die Abstützung der Antriebsfeder gegenüber dem Antriebsrotor und/oder dem Spannelement vorzugsweise indirekt, d.h. es sind weitere Bauelemente (beispielsweise Zahnräder oder andere Getriebeteile) vorgesehen, die die erforderliche Umsetzung des Kraftflusses gewährleisten. Der Begriff "Abstützen" ist also im allgemeinen Sinn dahingehend zu verstehen, dass eine Kraftübertragung zwischen dem Spannelement der Antriebsfeder und dem Antriebsrotor vorhanden ist, durch die die Drehung des Spannelementes bei fixiertem Antriebsrotor zum Spannen der Antriebsfeder führt und bei fixiertem Spannelement die Entspannungsbewegung der zuvor gespannten Feder den Antriebsrotor antreibt.
Aus der US 5,984,940 ist eine Lanzettenvorrichtung bekannt, bei der zum Spannen der Antriebsfeder ein Spannknopf um 180° gedreht wird, während der Antriebsrotor arretiert ist. Bei einer Einstichbewegung dreht sich der Antriebsrotor um 180°, während der Spannknopf arretiert ist.
Aus der WO 0100090 ist eine Lanzetten vorrichtung bekannt, bei der Spannen durch eine translatorische Bewegung erfolgt.

Die Erfindung wird nachfolgend anhand von in den Figuren dargestellten Ausführungsbeispielen näher erläutert. Die darin dargestellten Besonderheiten können einzeln oder in Kombination verwendet werden, um bevorzugte Ausgestaltungen der Erfindung zu schaffen. Es zeigen:
- Fig. 1: einen Längsschnitt eines erfindungsgemäßen Blutentnahmesystems;
- Fig. 2: eine teilweise aufgeschnittene Darstellung eines erfindungsgemäßen Blutentnahmesystems in fünf unterschiedlichen Bewegungspositionen bzw.-phasen (a) bis (e) des Lanzettenantriebs;
- Fig. 3: eine graphische Darstellung der Abhängigkeit der Einstichtiefe vom Drehwinkel des Antriebsrotors zur Erläuterung der Funktion unterschiedlicher Drehwinkelbereiche;
- Fig. 4: eine teilweise aufgeschnittene perspektivische Darstellung einer weiteren Ausführungsform eines erfindungsgemäßen Blutentnahmesystems;
- Fig. 5: eine perspektivische Darstellung des Antriebsmoduls des in dem Blutentnahmesystem von Figur 4 verwendeten Lanzettenantriebs;
- Fig. 6: eine perspektivische Explosionsdarstellung von Komponenten des Moduls von Figur 5;
- Fig. 7: eine perspektivische Darstellung eines Moduls gemäß Figur 5 in teilweise zusammengebautem Zustand;
- Fig. 8: eine perspektivische Darstellung eines Lanzettenhalters des Blutentnahmesystems gemäß Figur 4;
- Fig. 9: eine perspektivische Darstellung einer aus einem Antriebsmodul gemäß Figur 5 und einem Lanzettenhalter gemäß Figur 8 bestehenden Untereinheit des Blutlanzettensystems gemäß Figur 4.

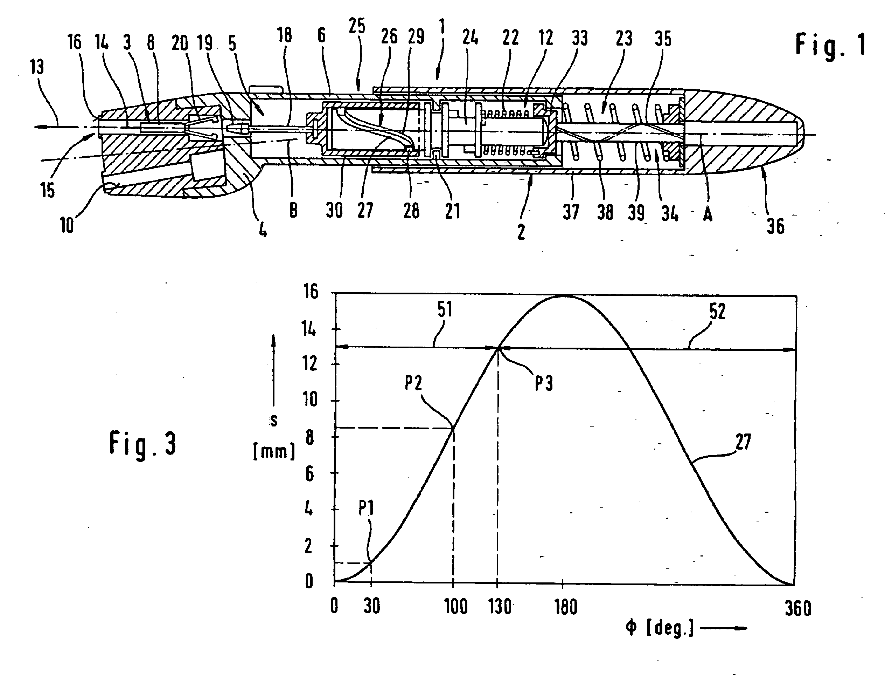

Das in Figur 1 dargestellte Blutentnahmesystem 1 besteht aus einem Stechgerät 2 und Lanzetten 3. Die Lanzetten 3 sind bei der dargestellten Ausführungsform in einem Revolverkopf 4 enthalten, der auswechselbar auf das vordere Ende 5 des Stechgerätes 2 als Teil von dessen Gehäuse 6 aufgesetzt werden kann.

Der Revolverkopf 4 ist um eine Rotationsachse B in mehrere Positionen drehbar, in denen jeweils eine Lanzette 3 koaxial mit der Hauptachse A des Stechgerätes 2 positioniert ist. Die Maße des Lanzettenkörpers 8 und der Ausnehmungen 9, in denen die Lanzetten 3 sitzen, sind so aufeinander abgestimmt, dass die Wände der Ausnehmungen 9 jeweils eine Lanzettenführung 10 bilden, durch die die Lanzette 3 auf einem vorbestimmten Einstichweg (hier längs der Hauptachse A) geführt wird.

In dem Gehäuse 6 des Stechgerätes 2 befindet sich ein Lanzettenanirieb 12, der dazu dient, jeweils eine Lanzette 3 mit hoher Geschwindigkeit in Einstichrichtung 13 zu bewegen, bis ihre Spitze 14 aus einer Austrittsöffnung 15 austritt, während das Stechgerät 2 mit einer die Austrittsöffnung 15 umgebenden Kontaktfläche 16 gegen ein nicht dargestelltes Körperteil gedrückt wird. Dadurch wird in dem Körperteil eine Wunde zur Entnahme von Blut erzeugt.

Um diese Einstichbewegung zu ermöglichen, muß jeweils eine Lanzette 3 mit dem Lanzettenantrieb 12 gekoppelt werden, bevor die Einstichbewegung ausgeführt wird. Dies geschieht bei der dargestellten Ausführungsform mittels einer als Stößel 18 bezeichneten Verbindungsstange. An dem der Lanzette 3 zugewandten Ende des Stößels 18 ist ein verdicktes Halteelement 19 vorgesehen, das zum Ankoppeln einer Lanzette in eine korrespondierende Haltevorrichtung 20 des Lanzettenkörpers 8 eingeführt wird. Die Haltevorrichtung 20 ist so ausgebildet, dass sie im Zusammenwirken mit der Formgebung der Ausnehmung 9 das Halteelement 19 des Stößels 18 umgreift, wenn der Stößel 18 so weit in Einstichrichtung 13 bewegt wird, dass sein vorderes Ende den Lanzettenkörper kontaktiert und die Lanzette 3 gegenüber der in Figur 1 dargestellten Position in Einstichrichtung (nach links) verschiebt. Dadurch wird die Lanzette 3 formschlüssig an den Lanzettenantrieb 12 angekoppelt. Nähere Einzelheiten und alternative Ausführungsformen eines geeigneten Kopplungsmechanismus sind in der internationalen Patentanmeldung PCT/EP01/12527 beschrieben. Deren Inhalt wird durch Bezugnahme zum Gegenstand der vorliegenden Anmeldung gemacht.

Bei der dargestellten bevorzugten Ausführungsform ist die Lanzette 3 "direkt geführt" d.h. sie befindet sich unmittelbar in einem die erforderliche Führung während der Einstichbewegung bildenden Teil des Gehäuses 2 (im vorliegenden Fall eines Magazins, das eine Mehrzahl von Lanzetten enthält). Die nachfolgend erläuterte erfindungsgemäße Ausführungsform des Lanzettenantriebs eignet sich in besonderem Maße für solche direkt geführten magazinierten Lanzetten. Sie ist jedoch auch mit den bisher überwiegend gebräuchlichen indirekten Lanzettenführungen verwendbar, bei denen der Lanzettenantrieb permanent mit einem Lanzettenhalter gekoppelt ist, in den manuell für jede Blutentnahme eine neue Lanzette eingesetzt wird. Während des Einstichvorganges wird der Lanzettenhalter mittels eines als Führung dienenden Gehäuseteils geführt und sorgt damit indirekt für die erforderliche Führung der Lanzette auf dem Einstichweg. Derartige Konstruktionen sind in den einleitend genannten Vorpublikationen beschrieben.

Der Lanzettenantrieb 12 besteht im wesentlichen aus einer Antriebsfeder 22, einer Spanneinrichtung 23 zum Spannen der Antriebsfeder 22 und einem von der Antriebsfeder 22 angetriebenen um die Achse A rotierenden Antriebsrotor 24, der durch einen Lagerzapfen 21 gegen axiale Verschiebung gesichert ist. Durch einen abtriebsseitigen Kopplungsmechanismus 25 wird die Drehbewegung des Antriebsrotors 24 in die Einstechbewegung umgesetzt, die über den Stößel 18 auf eine daran angekoppelte Lanzette 3 übertragen wird.

Der abtriebsseitige Kopplungsmechanismus 25 wird im dargestellten Fall von einer Kurvensteuerung mit einer Steuerkurve 27 und einem die Steuerkurve 27 während der Einstichbewegung abfahrenden Steuerzapfen 28 gebildet. Bei der dargestellten Ausführungsform wird die Steuerkurve 27 von einer am Umfang des Antriebsrotors 24 umlaufenden Ausnehmung gebildet. Der Steuerzapfen ist an einer Vortriebshülse 30 ausgebildet ist, die den mit der Steuerkurve 27 versehenen Teil des Antriebsrotors 24 umschließt. Die Vortriebshülse 30 ist durch eine nicht dargestellte Längsnut drehfest so geführt, dass sie nur eine Translationsbewegung ausführen kann. An ihrem vorderen Ende ist der Stößel 18 starr fixiert. Die Kurvensteuerung 26 funktioniert grundsätzlich in gleicher Weise, wie die in dem US-Patent 5,318,584 und in der EP 1034740 A1 beschriebenen Kurvensteuerungen. Ein wesentlicher Unterschied besteht jedoch darin, dass der Antriebsrotor während des Spannens der Antriebsfeder nicht zurückgedreht werden muß. Deshalb kann einerseits eine sehr einfache Gestaltung der Steuerkurve 27 gewählt und andererseits der gesamte Drehwinkel von 360° für die Umsetzung der Drehbewegung des Antriebsrotors 24 in eine Translationsbewegung des Stößels 18 und einer damit verbundenen Lanzette 3 genutzt werden.

Dies wird dadurch erreicht, dass die Spanneinrichtung 23 erfindungsgemäß gemäß dem OWADAC-Prinzip konstruiert ist. Das von dem Antriebsrotor 24 abgewandte Ende der Antriebsfeder 22 stützt sich gegen ein drehbewegliches Spannelement 33 ab, welches zum Spannen der Antriebsfeder 22 bei gehemmter Rotation des Antriebsrotors 24 in die gleiche Drehrichtung drehbar ist, in die sich der Antriebsrotor während der Vortriebsphase dreht. Während der Vortriebsphase ist das Spannelement 33 gegen eine Rückwärtsdrehung arretiert, so dass der Antriebsrotor nach Freigabe der seine Rotation verhindernden Hemmung die Drehbewegung durchführt, die in die Einstichbewegung der Lanzette umgesetzt wird.

Bei der in Figur 1 dargestellten Ausführungsform ist das drehbewegliche Spannelement 33 über ein Drehschiebegetriebe 34, das im dargestellten Fall wiederum mittels einer Steuerkurve 35 realisiert ist, mit einem translatorisch bewegbaren Betätigungselement 36 verbunden, das aus dem Gehäuse 6 herausragt. Das Betätigungselement 36 wird im dargestellten Fall von einer Schiebehülse 37 gebildet, die den (bezogen auf die Einstichrichtung 13) hinteren Teil des Gehäuses 6 bildet und gegen die Kraft einer Rückstellfeder 38 in Richtung der Hauptachse A des Stechgerätes 2 nach vorne verschoben werden kann. Die Steuerkurve 35 ist in einer Welle 39 ausgebildet, die mit dem Spannelement 33 drehfest verbunden ist. Sie ist so gelagert, dass sie mit dem Spannelement gedreht, aber nicht axial verschoben werden kann. Die Bewegung der Schiebehülse 37 wird mittels der Steuerkurve 35 und eines diese abfahrenden (in der Figur nicht dargestellten) Steuerzapfens in eine Drehbewegung des Spannelementes 33 umgesetzt.

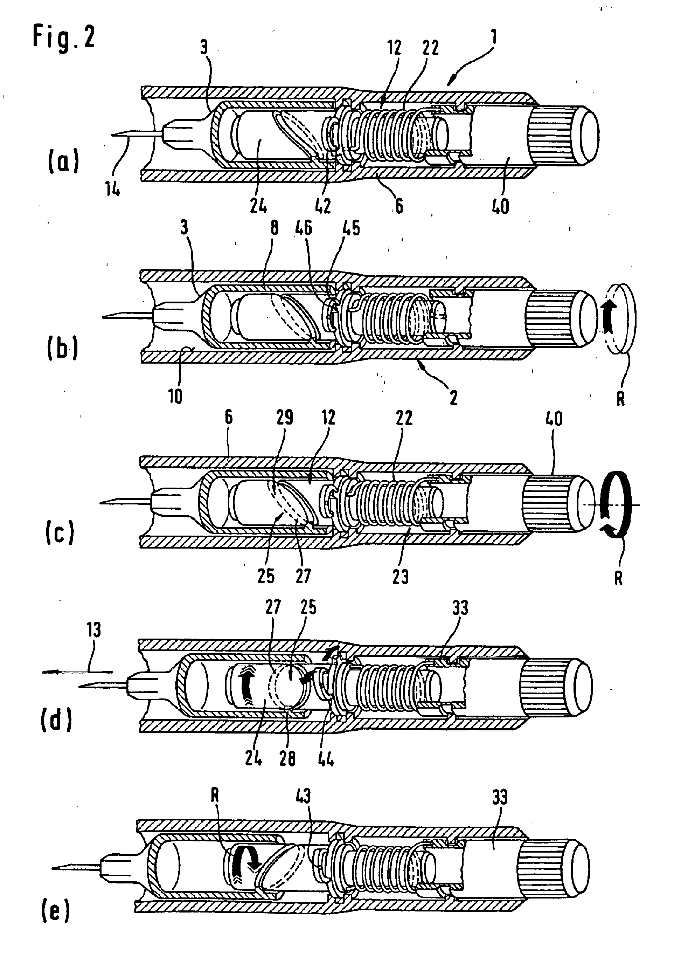

Die wesentlichen Funktionen des Lanzettenantriebs 12 lassen sich anhand von Figur 2 noch deutlicher erkennen. Sie zeigt eine alternative Ausführungsform eines erfindungsgemäßen Blutentnahmesystems 1 in fünf verschiedenen Bewegungsphasen. Funktionsgleiche Bauelemente sind mit den gleichen Bezugszeichen wie in Figur 1 bezeichnet. Es bestehen vor.allem folgende Unterschiede:
- Um eine möglichst einfache Darstellung und gute Erkennbarkeit der für die Erfindung wesentlichen Funktionen zu gewährleisten, wurde der in der Praxis übliche Mechanismus zum Ankoppeln auswechselbarer Lanzetten weggelassen. Stattdessen weist das in Figur 2 dargestellte Blutentnahmesystem eine mit der Vortriebshülse 30 fest verbundene Lanzette 3 auf.
- Zum Spannen des Lanzettenantriebs 12 dient ein aus dem hinteren Ende des Gehäuses 6 herausragendes drehbares Betätigungselement 40, mit dem das Spannelement 33 fest verbunden ist.
- Als konstruktives Element, durch das die erforderliche Hemmung der Rotation des Antriebsrotors während der Spannphase erreicht werden kann, dient bei der dargestellten bevorzugten Ausführungsform ein Sperrnocken 42, der durch Schwenken um eine Achse 43 in zwei verschiedene Positionen gebracht werden kann, bei denen sich jeweils eine der beiden an seinen Enden vorgesehenen Sperrklauen 44,45 im Bewegungsweg eines an dem Antriebsrotor 24 vorgesehenen Anschlagelementes 46 befindet. Die, bezogen auf die Drehrichtung R des Antriebsrotors 24, erste Sperrklaue wird als vordere Sperrklaue, die zweite als hintere Sperrklaue 45 bezeichnet.

Die in der Teilfigur (a) dargestellte Bewegungsposition entspricht dem Grundzustand des Lanzettenantriebs. Die Antriebsfeder 22 ist entspannt. Das Anschlagelement 46 liegt an der vorderen Sperrklaue 44 des Sperrnockens 42 an.

Wenn der Sperrnocken mittels eines nicht dargestellten Betätigungselementes in die in der Teilfigur (b) dargestellte Position geschwenkt wird, kann der Antriebsrotor 24 durch einen Drehwinkelbereich gedreht werden, der dem Abstand zwischen der vorderen Sperrklaue 44 und der hinteren Sperrklaue 45 des Sperrnockens 42 entspricht und der als Vorbereituhgs-Drehwinkelbereich bezeichnet wird. Diese Drehbewegung des Antriebsrotors 24 wird durch entsprechendes Drehen des Betätigungselementes 40 bewirkt, das über die Drehfeder 22 ein Drehmoment unmittelbar auf den Antriebsrotor 24 überträgt. Wenn die Antriebsfeder 22 in dem Grundzustand (a) vollständig entspannt war, werden die Bauelemente 40,22 und 24 gemeinsam gleichmäßig gedreht. Wenn hingegen die Antriebsfeder 22 in dem Grundzustand (a) noch unter einer Restspannung stand, erfolgt die Bewegung in dem Vorbereitungs-Drehwinkelbereich teilweise oder vollständig durch das von der Restspannung der Antriebsfeder 22 ausgeübte Drehmoment. In jedem Fall resultiert eine (im Vergleich zu der Einstichbewegung) langsame Drehbewegung des Antriebsrotors, die durch den abtriebsseitigen Kopplungsmechanismus 25 (hier die durch die Steuerkurve 27 und den Steuerzapfen 28 gebildete Kurvensteuerung 26) in eine verhältnismäßig langsame Bewegung der Lanzette 3 umgesetzt.

Die Bewegung des Lanzettenantriebs in dem Vorbereitungs-Drehwinkelbereich kann zur Vorbereitung des eigentlichen Einstichvorganges genutzt werden. Insbesondere kann er dazu dienen, den Lanzettenantrieb mit einer magazinierten Lanzette zu koppeln, wobei insbesondere der in Figur 1 dargestellte und in der PCT/EP01/12527 näher erläuterte Kopplungsmechanismus zur Anwendung kommen kann. Der Vorbereitungs-Drehwinkelbereich kann jedoch auch zu anderen Zwecken vorteilhaft genutzt werden, beispielsweise um einen Lanzettenhalter in eine Position zu bringen, in der eine verbrauchte Lanzette ausgeworfen wird und der Lanzettenhalter zur Aufnahme einer neuen Lanzette bereit ist.

Während der in der Teilfigur (c) dargestellten Spannphase des Lanzettenantriebs 12 liegt das Anschlagelement 46 an der hinteren Sperrklaue 45 des Sperrnockens 42 an. Dadurch ist die Rotation des Antriebsrotors 24 in Drehrichtung R gehemmt. Durch Drehen des Betätigungselementes 40 und des mit diesem gekoppelten drehbeweglichen Spannelementes 33 in die gleiche Drehrichtung R wird die Feder 22 gespannt. Am Ende der Spannbewegung rastet das Spannelement 33 mittels eines nicht dargestellten Rastmechanismus derart ein, dass es während der nachfolgenden Vortriebsphase gegen eine Rückwärtsdrehung arretiert ist.

Die in der Teilfigur (d) dargestellte Vortriebsphase des Lanzettenantriebs 12 wird dadurch ausgelöst, dass der Sperrnocken 42 in die Position geschwenkt wird, in der seine hinteren Sperrklaue 45 den Antriebsrotor 24 freigibt, während seine vordere Sperrklaue 44 in den Rotationsweg des Anschlagelementes 46 geschwenkt wird. Nach der Freigabe führt der Antriebsrotor 24, von der stark gespannten Antriebsfeder 22 getrieben, eine rasche Rotationsbewegung aus, die von dem abtriebsseitigen Kopplungsmechanismus 25 in eine präzise geführte und rasche (deshalb schmerzarme) Einstich- und Rückführbewegung der Lanzette umgesetzt wird.

Teilfigur (e) zeigt die Position der maximalen Eindringtiefe der Lanzette 3, die dem unteren Umlenkpunkt der Steuerkurve 27 entspricht. Am Ende der Einstich- und Rückführbewegung wird die Drehbewegung durch die vordere Sperrklaue 44 des Sperrnockens 42 gestoppt und der Lanzettenantrieb befindet sich in dem Grundzustand (a).

An Hand von Figur 3 soll zusätzlich verdeutlicht werden, in welcher Weise der beschriebene Lanzettenantrieb benutzt werden kann, um in zwei getrennten Drehwinkelbereichen unterschiedliche Funktionen zu realisieren. Die dargestellte Sinuskurve stellt eine Abwicklung einer Steuerkurve 27 in die Zeichenebene dar. Der gesamte Drehwinkelbereich des OWADAC-Antriebs (360°) ist in einen Vorbereitungs-Drehwinkelbereich 51 (im dargestellten Fall 130°) und in einen Einstich-Drehwinkelbereich 52 (230°) unterteilt.

Zu Beginn des Vorbereitungs-Drehwinkelbereiches 30 ist die Steigung der Steuerkurve 27 gering. Dies führt zu einer langsamen Bewegung mit relativ hoher Kraft. In der Position P1 (im dargestellten Fall bei einer Einstichtiefe von 1 mm und einem Winkel von 30°) wird mittels des vorderen Endes des Stößels 18 (Figur 1) eine Schutzfolie durchstoßen, die die Lanzettenaufnahme des Revolverkopfes 4 nach hinten abdeckt. Während der weiteren Bewegung trifft der Stößel bei der Steuerkurvenposition P2 (bei 100° und etwa 8,5 mm Bewegungsweg) auf das Ende der Lanzette 3. Das Halteelement 19 des Stößels 18 dringt in die Haltevorrichtung 20 der Lanzette 3 ein, wodurch die Lanzette 3 an den Länzettenantrieb 12 angekoppelt wird. Am Ende des Vorbereitungs-Drehwinkelbereiches 51 (Figur 2 (b)) befindet sich der Steuerzapfen 28 in der Position P3. Nach dem Spannen und Lösen der Hemmung dreht sich der Antriebsrotor 24 durch den Drehwinkelbereich 52, wobei die Einstich- und Rückführbewegung stattfindet.

Die Achse des Antriebsrotors muß nicht (wie in den Figuren 1 und 2 dargestellt) parallel zu der Einstichrichtung verlaufen. Alternativ kann auch ein Antriebsrotor verwendet werden, dessen Achse senkrecht zu der Einstichrichtung und der Hauptachse des Stechgerätes verläuft. In diesem Fall kann der abtriebsseitige Kopplungsmechanismus beispielsweise durch einen Pleuelantrieb (vgl. US-Patent 4,924,879) gebildet werden. Die Kraftübertragung von einem in Einstichrichtung translatorisch beweglichen Betätigungselement auf ein koaxial zu dem Antriebsrotor drehbares Spannelement kann beispielsweise mittels einer Zahnstange und eines mit dem Spannelement gekoppelten Ritzels erfolgen.

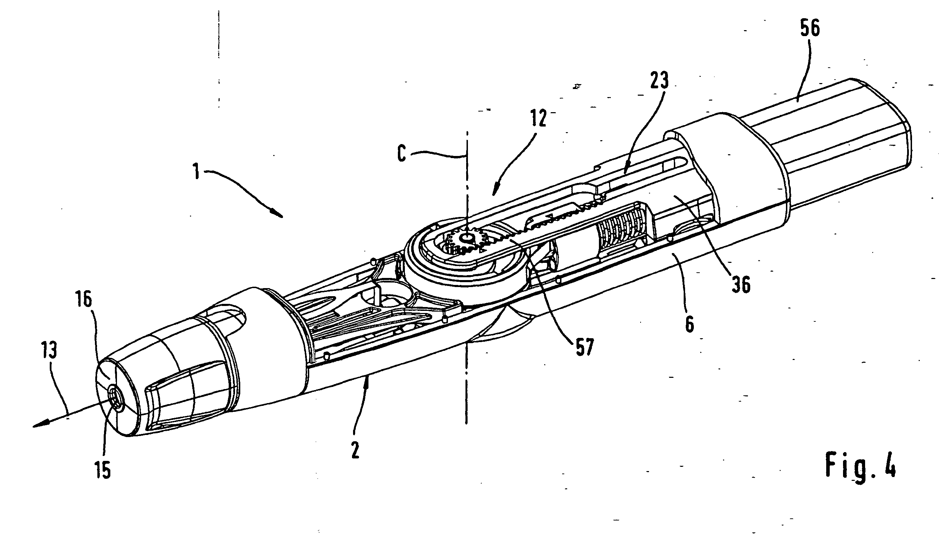

Eine derartige Ausführungsform eines Blutentnahmesystems 1 ist in den Figuren 4 bis 9 dargestellt. Das zentrale Element des Lanzettenantriebs 12 ist bei dieser Ausführungsform ein Antriebsmodul 55. Bestandteile des Antriebsmoduls 55 sind der Antriebsrotor 24 und das drehbewegliche Spannelement 33, die um eine gemeinsame Achse C drehbar sind, welche senkrecht zu der Einstichrichtung 13 und zu der Längsachse des Stechgerätes 2 verläuft.

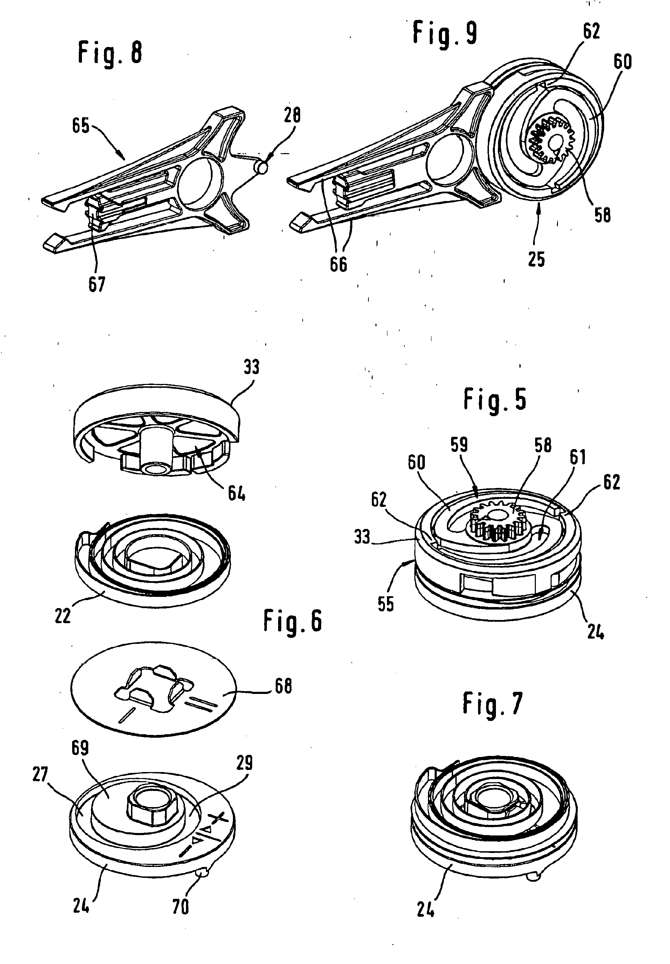

Zum Spannen des Lanzettenantriebs 12 wird ein translatorisch bewegliches Betätigungselement 36 mittels eines Betätigungsknopfes 56 in Einstichrichtung gedrückt. Eine Zahnstange 57 ist Bestandteil des Betätigungselementes 36 und treibt ein mit dem Spannelement 33 koaxiales Ritzel 58 an. Das Ritzel 58 ist mit dem Spannelement 33 über einen Freilauf 59 derart verbunden, dass beide Teile während der Spannbewegung (Bewegung des Betätigungselementes 36 in Einstichrichtung) miteinander gekoppelt, hingegen bei der Rückführbewegung des Betätigungselementes 36 entkoppelt sind. Bei der dargestellten Ausführungsform ist der Freilauf 59 mittels zweier elastischer Zungen 60 realisiert, die mit dem Ritzel 58 verbunden sind. Die Zungen 60 befinden sich in einer von dem Antriebsrotor 24 abgewandten Ausnehmung 61 des Spannelementes 33 mit Anschlägen 62, an denen die Enden der Zungen in der Kopplungs- Drehrichtung (in Figur 5 im Uhrzeigersinn) anliegen, während in der umgekehrten Richtung das Ritzel 58 relativ zu dem Spannelement 33 frei drehen kann.

Durch die Drehung des Spannelementes wird die Antriebsfeder 22 gespannt, die bei dieser Ausführungsform als Spiralfeder ausgebildet und in einer dem Antriebsrotor 24 zugewandten Ausnehmung 64 des drehbeweglichen Spannelementes 33 gelagert ist.

Auch bei dieser Ausführungsform schließt der abtriebsseitige Kopplungsmechanismus 25 eine Steuerkurve 27 ein, die von einer Ausnehmung 29 in dem Antriebsrotor 24 gebildet wird. Die Steuerkurve 27 hat im dargestellten Fall die Form eines zu der Achse C exzentrischen Kreises. Sie wird während der Drehung des Antriebsrotors 24 von einem Steuerzapfen 28 abgefahren, der Bestandteil eines Lanzettenhalters 65 ist. Zur Fixierung einer nicht dargestellten Lanzette hat der Lanzettenhalter 65 elastische Arme 66 und ein Anschlagelement 67, deren Formgebung so auf die korrespondierende Formgebung einer Lanzette angepaßt ist, dass diese in einer exakt reproduzierbaren Longitudinalposition in dem Halter 65 gehalten wird. Dieses Konstruktionsprinzip ist (beispielsweise aus dem US-Patent 5,318,584) bekannt und muß nicht näher erläutert werden.

Das hintere Ende des Lanzettenhalters 65 mit dem Steuerzapfen 28 greift in einen umlaufenden Schlitz des Antriebsmoduls 55 derartig ein, dass die Drehbewegungen der Bestandteile des Moduls 55 nicht behindert werden. Um dies sicherzustellen, ist bei der dargestellten Ausführungsform eine Trennscheibe 68 aus Metall vorgesehen, die auf einem Plateau 69 des Antriebsrotors 24 derartig aufliegt, dass zwischen ihr und den radial auswärts von der Steuerkurve 27 gelegenen Teilen des Rotors 24 ein umlaufender Spalt mit der erforderlichen Breite für den Lanzettenhalter 65 verbleibt.

Auch bei dieser Ausführungsform schließt der Spann- und Einstichvorgang die anhand der Figuren 1 bis 3 erläuterten Bewegungsphasen ein:
- Während des Spannens dreht sich das drehbewegliche Spannelement 33 in einer vorgegebenen Richtung (in Figur 4 im Uhrzeigersinn), wobei die Feder 22 gespannt wird, während die Rotation des Antriebsrotors 24 (mittels eines auf einen Sperrzapfen 70 wirkenden nicht näher dargestellten Auslösemechanismus) gehemmt ist.
- In einer Einstichphase treibt die Antriebsfeder 22 den Antriebsrotor 24 (nach Lösen der auf den Zapfen 70 wirkenden Hemmung) an, während gleichzeitig das Spannelement 33 (beispielsweise mittels einer in eine Ausnehmung des Spannelementes 33 eingreifenden nicht dargestellten elastischen Klinke) gegen eine Rückwärtsdrehung arretiert ist.

Aufgrund der vorliegenden Beschreibung sind dem Fachmann zahlreiche alternative Ausgestaltungen der Erfindung möglich. Beispielsweise können folgende Abwandlungen vorgenommen werden:
- Ein translatorisch bewegliches Betätigungselement 36 (Figur 1) kann selbstverständlich auch so konstruiert werden, dass der Lanzettenantrieb nicht durch eine Vorwärtsbewegung des Betätigungselementes (in Einstichrichtung 13), sondern durch eine Rückwärtsbewegun 19 (d.h. durch Ziehen statt Drücken) gespannt wird.
- Als Antriebsfeder 22 eignet sich grundsätzlich jedes drehelastische Federelement, also insbesondere eine Drehfeder, eine Torsionsfeder oder ein Torsionsstab.
- Bei der dargestellten Ausführungsform (Figur 2) ist die erforderliche Hemmung des Antriebsrotors 24 mit einem manuell betätigbaren Auslöser gekoppelt. Alternativ besteht jedoch auch die Möglichkeit, eine selbstlösende Hemmung zu verwenden, die die Drehbewegung des Antriebsrotors freigibt, wenn das von der Antriebsfeder 22 auf den Antriebsrotor 24 übertragene Drehmoment während des Spannvorganges einen definierten Wert überschreitet. In Verbindung mit einem in Einstichrichtung translatorischbeweglichen Betätigungselement für die Spannbewegung resultiert damit ein Lanzettenantrieb, bei dem der gesamte Bewegungsvorgang beim Drücken des Betätigungselementes automatisch abläuft.

## Patentansprüche

1. Blutentnahmesystem zur Entnahme von Blut für Diagnosezwecke, umfassend
ein Gehäuse (6), in dem eine Lanzette (3) entlang eines vorbestimmten Einstichweges beweglich ist,
eine Lanzettenführung (10), von der die Lanzette (3) auf dem vorbestimmten Einstichweg geführt wird, und
einen Lanzettenantrieb (12) mit einer Antriebsfeder (22), einer Spanneinrichtung (23), um in einer Spannphase des Lanzettenantriebs (12) die Antriebsfeder (22) zu spannen, einem von der Antriebsfeder (22) angetriebenen, um eine Achse (A) drehbaren Antriebsrotor (24) und einem abtriebsseitigen Kopplungsmechanismus (25), durch den die Drehbewegung des Antriebsrotors (24) in einer Vortriebsphase des Lanzettenantriebs (12) in die Einstichbewegung umgesetzt wird, bei der die Lanzette (3) mit hoher Geschwindigkeit in Einstichrichtung (13) bewegt wird, um eine Wunde zu erzeugen,
wobei
sich das von dem Antriebsrotor (24) abgewandte Ende der Antriebsfeder (22) gegen ein drehbewegliches Spannelement (33) abstützt,
das Spannelement (33) zum Spannen der Antriebsfeder (22) bei gehemmter Rotation des Antriebsrotors (24) in die gleiche Drehrichtung (R) drehbar ist, in die sich der Antriebsrotor (24) während der Vortriebsphase dreht,
das Spannelement (33) während der Vortriebsphase gegen eine Rückwärtsdrehung arretiert ist, so dass der Antriebsrotor (24) nach Freigabe der Hemmung eine Drehbewegung durchführt, die mittels des abtriebsseitigen Kopplungsmechanismus in die Einstichbewegung der Lanzette umgesetzt wird, **dadurch gekennzeichnet, daß** das Spannelement (33) mit einem translatorisch bewegbaren Betätigungselement (36) gekoppelt ist, und
die Hemmung der Drehbewegung des Antriebsrotors (24) selbst- , lösend ist, so dass das Spannen der Antriebsfeder (22) und die Freigabe der Drehbewegung des Antriebsrotors (24) beim Drücken des Betätigungselementes (36) automatisch abläuft.

2. Blutentnahmesystem nach Anspruch 1, **dadurch gekennzeichnet, dass** die Drehachse des Spannelementes (33) parallel, vorzugsweise koaxial zu der Drehachse (A) des Antriebsrotors (24) verläuft.

3. Blutentnahmesystem nach einem der vorhergehenden Ansprüche, **dadurch gekennzeichnet, dass** das Spannelement (33) über ein Drehschiebegetriebe (34) mit dem translatorisch bewegbaren Betätigungselement (36) gekoppelt ist.

4. Blutentnahmesystem nach einem der vorhergehenden Ansprüche, **dadurch gekennzeichnet, dass** die Rotationsachse (C) des Spannelementes (33) quer zur Bewegungsrichtung das translatorisch bewegbaren Betätigungselementes (36) verläuft.

5. Blutentnahmesystem nach Anspruch 4, **dadurch gekennzeichnet, dass** das Betätigungselement (36) mittels einer Zahnstange (57) auf ein während der Spannbewegung mit dem Spannelement (33) gekoppeltes Ritzel (58) wirkt.

6. Blutentnahmesystem nach Anspruch 5, **dadurch gekennzeichnet, dass** das Ritzel, das drehbewegliche Spannelement (33) und der Antriebsrotor (24) um eine gemeinsame Achse (C) drehbare Bestandteile eines Antriebsmoduls (55) sind.

7. Blutentnahmesystem nach einem der vorhergehenden Ansprüche, **dadurch gekennzeichnet, dass** der Rotations-Drehwinkelbereich des Antriebsrotors einen Vorbereitungs-Drehwinkelbereich (51) und einen Einstich-Drehwinkelbereich (52) einschließt, die Rotationsbewegung des Antriebsrotors in dem VorbereitungsDrehwinkelbereich (51) seines Rotationsweges langsamer als in dem Einstich-Drehwinkelbereich (52) ist und die Drehbewegung des Antriebsrotors (24) am Übergang zwischen dem Vorbereitungs-Drehwinkelbereich (51) und dem Einstich-Drehwinkelbereich (52) derartig gehemmt ist, dass die Antriebsfeder (22) bei der weiteren Drehung des Spannelementes (33) in einen für die rasche Einstichbewegung erforderlichen Spannungszustand gebracht wird.

8. Blutentnahmesystem nach einem der vorhergegangenen Ansprüche, **dadurch gekennzeichnet, dass** die Hemmung der Drehbewegung des Antriebsrotors (24) derartig selbstlösend ist, dass der Antriebsrotor (24) freigegeben wird, wenn der für die Einstichbewegung erforderliche Spannungszustand der Antriebsfeder (22) erreicht ist.

9. Blutentnahmesystem nach einem der vorhergehenden Ansprüche, **dadurch gekennzeichnet, dass** der abtriebsseitige Koppiungsmechanismus (25) eine eine Steuerkurve (27) bildende Ausnehmung (29) einschließt, in die ein Steuerzapfen (28) eingreift, wobei während der in der Vortriebsphase stattfindenden Drehbewegung des Antriebsrotors der Steuerzapfen (28) die durch die Ausnehmung (29) gebildete Steuerkurve (27) abfährt und dadurch mindestens ein Teil der Einstich- und Rückführbewegung bestimmt wird.

10. Blutentnahmesystem nach Anspruch 9, **dadurch gekennzeichne**t, dass die die Steuerkurve (27) bildende Ausnehmung (29) in dem Antriebsrotor (24) ausgebildet ist.

11. Blutentnahmesystem nach Anspruch 10 in Verbindung mit Anspruch 5, **dadurch gekennzeichnet, dass** die Steuerkurve (27) bildende Ausnehmung (29) innerhalb des Antriebsmoduls (55) auf der dem Spannelement (33) zugewandten Seite des Antriebsrotors (24) verläuft.

## Claims

1. Blood removal system for removing blood for diagnostic purposes, including
a housing (6), in which a lancet (3) is moveable along a predetermined puncture path,
a lancet guide (10), by which the lancet (3) is guided on the predetermined puncture path, and
a lancet drive (12) with a drive spring (22), a cocking device (23) for tensioning the drive spring (22) in a cocking phase of the lancet drive (12), a drive rotor (24) which is driven by the drive spring (22) and which is rotatable about an axis (A), and an output-side coupling mechanism (25) for converting the rotational movement of the drive rotor (24) in a driving phase of the lancet drive (12) into a puncture movement wherein the lancet (3) is moved with high speed in a puncture direction (13) in order to produce a wound,
wherein
the end of the drive spring (22) that faces away from the drive rotor (24) is connected to a rotationally moveable cocking element (33),
the cocking element (33) is, with inhibited rotation of the drive rotor (24), rotatable for tensioning of the drive spring (22) in the same direction of rotation (R) in which the drive rotor (24) rotates during the driving phase, and
the cocking element (33) is arrested during the driving phase against a reverse rotation, so that the drive rotor (24), after release of the rotation inhibition, performs a rotational movement which is converted by means of the output-side coupling mechanism into the puncturing movement of the lancet, **characterized in that**
the cocking element (33) is coupled with a translatory moveable actuation element (36), and
the inhibition of the rotational movement of the drive rotor (24) is self-releasing, such that the tensioning of the drive spring (22) and the release of the rotational movement of the drive rotor (24) take place automatically when the actuation element (36) is pressed.

2. Blood removal system according to claim 1, **characterized in that** the axis of rotation of the cocking element (33) runs parallel, preferably coaxial, to the axis of rotation (A) of the drive rotor (24).

3. Blood removal system according to any one of the preceding claims, **characterized in that** the cocking element (33) is coupled by a rotary/sliding transmission (34) with the translatory moveable actuation element (36).

4. Blood removal system according to any one of the preceding claims, **characterized in that** the axis of rotation (C) of the cocking element (33) runs transverse to a direction of movement of a translatory moveable actuation element (36).

5. Blood removal system according to claim 4, **characterized in that** the actuation element (36) operates by means of a gear rod (57) on a pinion gear (58) that is coupled with the cocking element (33) during the cocking movement.

6. Blood removal system according to claim 5, **characterized in that** the pinion gear, the rotatably moveable cocking element (33) and the drive rotor (24) are components of a drive module (55) in which they are rotatable about a common axis (C).

7. Blood removal system according to any one of the preceding claims, **characterized in that** an angle of rotation range of the drive rotor includes a preparation angle of rotation range (51) and a puncture angle of rotation range (52), wherein the rotational movement of the drive rotor in the preparation angle of rotation range (51) is slower than in the puncture angle of rotation range (52), and the rotational movement of the drive rotor (24) is inhibited at the transition between the preparation angle of rotation range (51) and the puncture angle of rotation range (52) such that the drive spring (22), upon further rotation of the cocking element (33), is brought into a tensioned state required for the quick puncture movement.

8. Blood removal system according to any one of the preceding claims, **characterized in that** the inhibition of the rotational movement of the drive rotor (24) is self-releasing, such that the drive rotor (24) is released when the drive spring (22) is in a tensioned state required for the puncture movement.

9. Blood removal system according to any one of the preceding claims, **characterized in that** the output-side coupling mechanism (25) includes a recess (29) forming a control curve (27), wherein a control pin (28) engages in the recess (29), wherein during the rotational movement of the drive rotor occurring in the driving phase the control pin (28) travels along the control curve (27) formed by the recess (29), thereby determining at least a part of the puncturing and reverse movement.

10. Blood removal system according to claim 9, **characterized in that** the recess (29) forming the control curve (27) is provided in the drive rotor (24).

11. Blood removal system according to claim 10 in combination with claim 5, **characterized in that** the recess (29) forming the control curve (27) within the drive module (55) runs on a side of the drive rotor (24) facing the cocking element (33).

## Revendications

1. Système de prélèvement sanguin destiné à prélever du sang à des fins de diagnostic, comprenant
un boîtier (6) dans lequel une lancette (3) est mobile le long d'une trajectoire de piqûre prédéterminée,
un guide de lancette (10) par lequel la lancette (3) est guidée sur la trajectoire de piqûre prédéterminée et
un entraînement de lancette (12) comprenant un ressort de commande (22), un dispositif tendeur (23) destiné à tendre le ressort de commande (22) dans une phase de tension de l'entraînement de lancette (12), un rotor d'entraînement (24) commandé par le ressort de commande (22) et pouvant tourner autour d'un axe (A) et un mécanisme de couplage (25) monté côté sortie, grâce auquel, dans une phase de propulsion de l'entraînement de lancette (12), le mouvement de rotation du rotor d'entraînement (24) est transformé en mouvement de piqûre lors duquel la lancette (3) est déplacée à grande vitesse dans le sens de piqûre (13) afin de former une plaie,
l'extrémité du ressort de commande (22) opposée au rotor d'entraînement (24) s'appuyant contre un élément tendeur (33) mobile en rotation, l'élément tendeur (33) destiné à tendre le ressort de commande (22) pouvant en cas de rotation bloquée du rotor d'entraînement (24) être tourné dans le même sens de rotation, dans lequel tourne le rotor d'entraînement (24) pendant la phase de propulsion,
l'élément tendeur (33) étant bloqué pendant la phase de propulsion contre une rotation arrière, de sorte que le rotor d'entraînement (24), après libération du blocage, effectue un mouvement de rotation qui est transformé au moyen du mécanisme de couplage monté côté sortie, en mouvement de piqûre de la lancette, **caractérisé en ce que** l'élément tendeur (33) est couplé à un élément actionneur (36) mobile en translation, et le blocage du mouvement de rotation du rotor d'entraînement (24) se libère automatiquement, de sorte que les opérations de tension du ressort de commande (22) et de libération du mouvement de rotation du rotor d'entraînement (24) se déroulent automatiquement en poussant l'élément actionneur (36).

2. Système de prélèvement sanguin selon la revendication 1, **caractérisé en ce que** l'axe de rotation de l'élément tendeur (33) s'étend parallèlement, de préférence coaxialement à l'axe de rotation (A) du rotor d'entraînement (24).

3. Système de prélèvement sanguin selon l'une des revendications précédentes, **caractérisé en ce que** l'élément tendeur (33) est couplé à l'élément actionneur (36) mobile en translation, par l'intermédiaire d'un mécanisme rotatif coulissant (34).

4. Système de prélèvement sanguin selon l'une des revendications précédentes, **caractérisé en ce que** l'axe de rotation (C) de l'élément tendeur (33) s'étend perpendiculairement au sens de déplacement de l'élément actionneur (36) mobile en translation.

5. Système de prélèvement sanguin selon la revendication 4, **caractérisé en ce que** l'élément actionneur (36) agit au moyen d'une crémaillère (57) sur un pignon (58), couplé à l'élément tendeur (33) pendant le mouvement de tension.

6. Système de prélèvement sanguin selon la revendication 5, **caractérisé en ce que** ledit pignon, l'élément tendeur (33) mobile en rotation et le rotor d'entraînement (24) sont des parties constitutives d'un module d'entraînement (55), pouvant tourner autour d'un axe commun (C).

7. Système de prélèvement sanguin selon l'une des revendications précédentes, **caractérisé en ce que** la plage angulaire de rotation du rotor d'entraînement comprend une plage angulaire de préparation (51) et une plage angulaire de piqûre (52), le mouvement de rotation du rotor d'entraînement est plus lent dans la plage angulaire de préparation (51) de sa trajectoire de rotation que dans la plage angulaire de piqûre (52) et au niveau de la transition entre la plage angulaire de préparation (51) et la plage angulaire de piqûre (52) le mouvement de rotation du rotor d'entraînement (24) est bloqué, de telle sorte qu'à la rotation suivante de l'élément tendeur (33), le ressort de commande (22) est mis en état de contrainte nécessaire pour le mouvement de piqûre rapide.

8. Système de prélèvement sanguin selon l'une des revendications précédentes, **caractérisé en ce que** le blocage du mouvement de rotation du rotor d'entraînement (24) se libère automatiquement, de telle sorte que le rotor d'entraînement (24) est libéré lorsque l'état de contrainte nécessaire pour le mouvement de piqûre du ressort de commande (22) est atteint.

9. Système de prélèvement sanguin selon l'une des revendications précédentes, **caractérisé en ce que** le mécanisme de couplage (25) monté côté sortie comprend un creux (29) formant une came de commande (27), creux dans lequel s'engage un tourillon de commande (28), dans lequel pendant le mouvement de rotation du rotor d'entraînement qui a lieu dans la phase de propulsion, le tourillon de commande (28) descend la came de commande (27) formée par le creux (29), permettant ainsi de déterminer au moins une partie du mouvement de piqûre et du mouvement de retour.

10. Système de prélèvement sanguin selon la revendication 9, **caractérisé en ce que** le creux (29) formant la came de commande (27) est ménagé dans le rotor d'entraînement (24).

11. Système de prélèvement sanguin selon la revendication 10 combinée avec la revendication 5, **caractérisé en ce que** le creux (29) formant la came de commande (27) s'étend à l'intérieur du module d'entraînement (55) sur le côté tourné vers l'élément tendeur (33) du rotor d'entraînement.
